(19) **Europäisches Patentamt**

**European Patent Office**

**Office européen des brevets**

(11) **EP 0 708 074 A1**

(12) **EUROPEAN PATENT APPLICATION**

(43) Date of publication:
24.04.1996 Bulletin 1996/17

(51) Int. Cl.⁶: **C07C 63/313**, C07C 51/265, C07C 15/02, C07C 2/66

(21) Application number: 95116565.3

(22) Date of filing: 20.10.1995

(84) Designated Contracting States:
BE DE FR GB NL

(30) Priority: 21.10.1994 JP 281513/94

(71) Applicant: MITSUBISHI OIL CO., LTD.
Minato-ku Tokyo (JP)

(72) Inventors:
• Aizono, Hirofumi
Yokohama-shi, Kanagawa (JP)

• Kouchi, Takeshi,
CII-212 Guriin Hiru Fujigaoka
Yokohama-shi, Kanagawa (JP)

(74) Representative: Hansen, Bernd, Dr. Dipl.-Chem. et al
Hoffmann, Eitle & Partner,
Patentanwälte,
Arabellastrasse 4
D-81925 München (DE)

(54) **Process for the preparation of pyromellitic anhydride**

(57) A process for the efficient production of high purity pyromellitic anhydride from 1-ethyl-2,4,5-triisopropylbenzene as a starting material.

## Description

FIELD OF THE INVENTION

The present invention relates to a process for the preparation of pyromellitic anhydride. More particularly, the present invention relates to a process for the preparation of pyromellitic anhydride, comprising the step of oxidizing 1-ethyl-2,4,5-triisopropylbenzene as a starting material. Pyromellitic acid or its anhydride has found wide application as a starting material for plasticizers or heat-resistant high molecular weight compounds, or as a curing agent for epoxy resins, and thus is useful. It is therefore desirable to provide pyromellitic anhydride at a low cost. The present invention provides a process for the efficient preparation of this useful compound.

BACKGROUND OF THE INVENTION

Durene (1,2,4,5-tetramethylbenzene) is suitable as a starting material for producing pyromellitic anhydride. Many methods have been used which comprise the oxidation of durene to easily obtain pyromellitic anhydride such as those disclosed in JP-B-42-1008 (The term "JP-B" as used herein means an "examined Japanese patent publication"), and JP-B-45-15018. Examples of the method for the preparation of durene include (1) separation from the heavy components of reformed gasoline (C10 reformate), (2) disproportionation of 1,2,4-trimethylbenzene, (3) methylation of xylene, and (4) preparation from methanol as a starting material (referred to as the MTG method). However, in the case of separating durene from the heavy components of reformed gasoline, isodurene having a boiling point close to that of durene is produced in about the same amount as durene. The separation of durene from isodurene requires sophisticated separation techniques such as pressure crystallization. Further, the product thus separated has too low a durene content to be economical. There are problems in disproportionation of 1,2,4-trimethylbenzene with a purification process similar to that noted with reformed gasoline so this process is also not economical. If the methylation of xylene (3) is used to prepare durene as a starting material, 1,2,3,5-tetramethylbenzene, an isomer of durene, is produced as a by-product in large amounts. Thus, this method is inefficient. If a zeolite catalyst having a shape selectivity is used in the preparation of durene, the selectivity of 1,2,4,5-tetramethylbenzene can be enhanced. However, the catalyst life is short, and the yield is normally poor. The use of methanol as a starting material is not economical because of the high cost of methanol. In other words, durene is the most suitable starting material for producing pyromellitic anhydride. However, some problems arise in producing a high purity durene economically.

Pyromellitic anhydride can also be produced by using a durene type tetraalkylbenzene (1,2,4,5-tetraalkylbenzene). For example, 1,2,4,5-dimethyldiisopropylbenzene may be oxidized to obtain pyromellitic anhydride (as disclosed in JP-B-49-31973, JP-A-62-201645 (The term "JP-A" as used herein means an "unexamined published Japanese patent application")). Alternatively, pyromellitic anhydride can be prepared from diethyldiisopropylbenzene or triethylisopropylbenzene obtained from the isopropylation of diethylbenzene or triethylbenzene (as disclosed in JP-A-3-284645). However, all of these methods necessarily produce isomers such as 1,2,3,5-tetraalkylbenzene as by-products in addition to 1,2,4,5-tetraalkylbenzene as a starting material for pyromellitic anhydride. These by-products cause such problems as (1) reduction in the purity of the resulting pyromellitic anhydride and (2) reduction of the life and activity of the catalyst used in the oxidation reaction. Thus, these by-products are economically undesirable. Accordingly, there is a need for a durene type tetraalkylbenzene having a purity of at least 95 % by weight.

SUMMARY OF THE INVENTION

The present invention provides a process for the efficient preparation of pyromellitic anhydride from a high purity durene type tetraalkylbenzene.

More specifically, the extensive studies on the foregoing problems in the preparation of pyromellitic anhydride have been made. As a result, it was found that pyromellitic anhydride can be efficiently prepared from 1-ethyl-2,4,5-triisopropylbenzene, as a starting material, which has in turn been synthesized from ethylbenzene and propylene as starting materials. It was found that since 1-ethyl-2,4,5-triisopropylbenzene can be prepared from ethylbenzene and propylene as starting materials at a high selectivity, little or no isomers which cause difficulties in oxidation are produced, thus making it possible to efficiently prepare pyromellitic anhydride from 1-ethyl-2,4,5-triisopropylbenzene as a starting material.

1,2,4,5-tetraalkylbenzene can be prepared by alkylating a monocyclic aromatic group having 0 to 3 alkyl groups substituted on benzene with alcohol or olefin. In the present invention, ethylbenzene, which is a monoalkylbenzene, is used as a monocyclic aromatic group. Propylene is used as an alkylating agent. The resulting 1-ethyl-2,4,5-triisopropylbenzene is then used as a starting material to prepare pyromellitic anhydride. If an alkylation product of a monoalkylbenzene other than ethylbenzene, e.g., an alkylation product of toluene or cumene (isopropylbenzene), is used as a starting material, pyromellitic anhydride cannot be produced as efficiently. In other words, if toluene, which has a methyl group, is used, the yield of tetraalkylbenzene is satisfactory, but the selectivity for 1,2,4,5-tetraalkylbenzene is low, making it impossible to obtain high purity pyromellitic anhydride. If cumene is used, the isopropyl group causes steric hindrance that reduces the yield of tetraalkylbenzene, making it impossible to produce pyromellitic anhydride efficiently. When a dialkylbenzene such as xylene is alkylated, isomers of 1,2,4,5-tetraalkylbenzene are produced as by-products. Thus, the production of a high purity pyromellitic anhydride by an oxidation reaction cannot be accomplished unless a high degree of purification is conducted.

1-ethyl-2,4,5-trialkylbenzene can be obtained by alkylating ethylbenzene with olefin or alcohol. As the alcohol, methanol may be used. As the olefin, ethylene, propylene, 1-butene, cis 2-butene, trans 2-butene, 1-pentene, isopentene or the like may be used. Any of these alkylating agents can yield 1-ethyl-2,4,5-trialkylbenzene highly selectively. However, propylene provides a comparatively higher yield. Thus, propylene is particularly preferred as a starting material.

The alkylation reaction of ethylbenzene with propylene for the preparation of 1-ethyl-2,4,5-triisopropylbenzene can be accomplished by known catalysts. Examples of such catalysts include solid acid catalysts such as silica alumina and zeolite (JP-A-48-85540); cation exchange resins (JP-A-48-19526); and supported heteropolyacid and/or heteropolyacid salt catalysts (JP-A-05-9135).

All of these methods can provide the desired 1-ethyl-2,4,5-triisopropylbenzene. The solid acid catalysts such as silica alumina and zeolite can provide a satisfactory yield of ethyltriisopropylbenzene but are disadvantageous in that they have a short catalyst life and thus are impractical. The cation exchange resins have a low thermal stability and thus are impractical. Thus, supported heteropolyacid and/or heteropolyacid salt catalysts are preferred.

Specific examples of such heteropolyacids or heteropolyacid salts include dodecatungstophosphoric acid ($H_3PW_{12}O_{40}$), dodecatungstosilicic acid ($H_4SiW_{12}O_{40}$), dodecamolybdophosphoric acid ($H_3PMo_{12}O_{40}$), dodecatungstogermanic acid ($H_3GeW_{12}O_{40}$), and dodecamolybdogermanic acid ($H_3GeMo_{12}O_{40}$). Examples of the heteropolyacid salts include those obtained by substituting some or all of the hydrogen atoms in the foregoing heteropolyacids by alkali metals, alkaline earth metals, transition metals or amines. Silica gel, titania, activated carbon or the like may be used as carrier for the catalyst. The catalyst can be prepared by an impregnation method. The supported amount of the heteropolyacid is preferably from 1 to 50 % by weight, most preferably from 10 to 40 % by weight based on the total weight of the catalyst.

The alkylation reaction of ethylbenzene with propylene may be effected by either a continuous or batch reaction apparatus. Referring to the reaction conditions, the molar ratio of propylene/ethylbenzene as starting materials is preferably from 1.0 to 6.0, most preferably from 3.0 to 4.0; the reaction temperature is preferably from 70 °C to 200 °C, most preferably from 100 °C to 190 °C; and the reaction pressure is preferably between normal atmospheric pressure to 10 kg/cm$^2$, most preferably from 2 to 8 kg/cm$^2$. In the case where a continuous reaction apparatus is used, the starting materials are preferably supplied into the reaction zone at a weight hourly space velocity (WHSV) of from 0.1 to 10 hr$^{-1}$, most preferably from 0.5 to 6.0 hr$^{-1}$.

The main product thus obtained is triisopropylethylbenzene. The reaction solution contains starting materials and by-products such as monoisopropylethylbenzene, diisopropylethylbenzene and tetraisopropylethylbenzene depending on the reaction conditions. Triisopropylethylbenzene contains a small amount of isomers besides 1-ethyl-2,4,5-triisopropylbenzene. This product is then subjected to a conventional separation method to remove the catalyst and other undesirable substances. The product is then purified by distillation. The distillation may be effected under any conditions effective to separate triisopropylethylbenzene from other fractions. The distillation, if it is conducted at normal pressure, may be effected in a boiling range of from 130 °C to 300 °C, preferably from 180 °C to 290 °C. After distillation, the product can be further purified by crystallization to enhance the purity of 1-ethyl-2,4,5-triisopropylbenzene. Thus, crystallization is suitable for producing a high purity pyromellitic anhydride.

The production of pyromellitic anhydride by oxidation of the 1-ethyl-2,4,5-triisopropylbenzene thus obtained may be accomplished by liquid phase air oxidation, gas phase air oxidation, nitric acid oxidation, chromic acid oxidation, etc. The gas phase air oxidation method is preferred because of economy and simplicity of the apparatus. The gas phase air oxidation method may be accomplished by the ordinary durene type tetraalkylbenzene oxidation method (JP-B-42-15926, JP-B-45-4978).

An ordinary vanadium pentoxide catalyst ("Sekiyu Gakkaishi", vol. 9, No. 6, (1966), page 453, Ota et al., "Sekiyu Gakkaishi", vol. 13, No. 12, (1970), page 938, Wakita et al., "Sekiyu to sekiyu kagaku (Petroleum and Petrochemistry)", vol. 15, No. 4, (1971), page 39, Iwamoto) as the catalyst in the gas phase air oxidation method may be used. Vanadium pentoxide may be used alone or in the form of a multiple system such as a mixture, a compound or a complex. To the vanadium pentoxide as a base may be added potassium sulfate, sodium oxide, titanium oxide, phosphorus pentoxide, tungstic acid, tin oxide, molybdic acid, copper oxide, niobium pentoxide, selenium dioxide or the like to provide an improved catalyst. A catalyst obtained by supporting such a catalyst on a carrier for an oxidation catalyst such as molten alumina (alundum), silicon carbide (carborundum) and titanium oxide having a low surface area and a low porosity can be used to effect gas phase air oxidation.

The gas phase air oxidation reaction may be effected by any known method. A fixed bed or fluidized bed reaction apparatus may be used. The reaction is effected under reduced or raised pressure. As the oxygen source, air is effective. Alternatively, a mixture of oxygen and diluent gas such as nitrogen gas and carbon dioxide or air enriched with oxygen may be used. The oxygen source and 1-ethyl-2,4,5-triisopropylbenzene as a starting material are pre-heated to a temperature of from 100 °C to 200 °C before introduction into the reaction vessel. The amount of the oxidizing agent to be used for 1-ethyl-2,4,5-triisopropylbenzene should be not less than the stoichiometric amount. If the oxidizing agent is air, it is preferably used such that the amount of oxygen is from 100 to 1,000 mol, more preferably from 200 to 600 mol per 1 mol of 1-ethyl-2,4,5-triisopropylbenzene. The reaction temperature is normally from 300 °C to 600 °C, more preferably from 350 °C to 500 °C depending on the contact time as is commonly understood by those of skill in the art. The starting material gas thus heated is then supplied into the reaction zone at a space velocity of from 2,000 to 10,000 hr$^{-1}$.

The starting materials obtained according to the present invention can be subjected to a gas phase air oxidation reaction in the optimum ranges as herein described to obtain the desired pyromellitic anhydride at a high selectivity and in a high yield. The separation and production of pyromellitic anhydride from the reaction gas can be accomplished by any known method (e.g., separation/recovery of pyromellitic anhydride from water or a proper organic solvent, followed by purification of pyromellitic anhydride by crystallization from the solvent) to obtain a high purity pyromellitic anhydride.

In accordance with the present invention, an industrially favorable high purity pyromellitic anhydride can be obtained at a high efficiency by the use of 1-ethyl-2,4,5-triisopropylbenzene prepared from ethylbenzene and propylene as starting materials.

The present invention will be further described in the following examples, but the present invention should not be construed as being limited thereto. The selectivity of 1-ethyl-2,4,5-triisopropylbenzene is defined as follows:

$$\text{Selectivity (mol \%)} = [(\text{number of mol of 1-ethyl-2,4,5-triisopropylbenzene}$$
$$\text{produced})/(\text{number of mol of ethyltriisopropylbenzene produced})] \times 100.$$

## EXAMPLE 1

### (1) Preparation of Ethyltriisopropylbenzene

Propylene and ethylbenzene as starting materials (propylene/ethylbenzene molar ratio: 4.0) were supplied into and reacted in a fixed bed reactor at a reaction pressure of 4.5 kg/cm$^2$, a reaction temperature of 127 °C and WHSV of 2.0 hr$^{-1}$ in the presence of a catalyst comprising 20 % by weight of dodecatungstophosphoric acid supported on silica gel. The reaction product was then subjected to quantitative analysis by gas chromatography. As a result, a mixture comprising 56.1 mol % of 1-ethyl-2,4,5-triisopropylbenzene, 2.5 mol % of 1,2,3,5-ethyltriisopropylbenzene, 3.5 mol % of ethylbenzene, and 22.3 mol % of reaction intermediates such as ethylisopropylbenzene and 15.6 mol % of heavy fractions was obtained. The selectivity of 1-ethyl-2,4,5-triisopropylbenzene was 95.7 %. The reaction product was then subjected to distillation at normal pressure in a boiling range of from 170 °C to 250 °C by means of an apparatus having 30 theoretical stages. The distillate was then subjected to quantitative analysis by gas chromatography. As a result, a mixture comprising 91.7 mol % of 1-ethyl-2,4,5-triisopropylbenzene and 4.2 mol % of 1,2,3,5-ethyltriisopropylbenzene was obtained.

### (2) Oxidation of Ethyltriisopropylbenzene

The distillate thus obtained in the above noted process (1) was used to prepare pyromellitic anhydride. The reaction was effected at a reaction temperature of 450 °C, an air/1-ethyl-2,4,5-triisopropylbenzene molar ratio of 460 and a SV of 4,000 hr$^{-1}$ in the presence of a catalyst comprising vanadium pentoxide and phosphorus pentoxide (weight ratio: 100 : 10 : 1.5) supported on alundum. As a result, pyromellitic anhydride was obtained in a yield of 57.8 % with respect to 1-ethyl-2,4,5-triisopropylbenzene in the reaction product. The purity of pyromellitic anhydride was 95.2 %.

### EXAMPLE 2

### (1) Preparation of High Purity Ethyltriisopropylbenzene

The distillate obtained in the above noted process (1) of Example 1 was crystallized at a temperature of from - 30 °C to - 10 °C, and then filtered under a pressure of 100 kgf/cm$^2$ for 0.5 hour. The purity and yield of 1-ethyl-2,4,5-triisopropylbenzene are set forth in Table 1. The yield is the ratio of the amount of 1-ethyl-2,4,5-triisopropylbenzene to that of 1-ethyl-2,4,5-triisopropylbenzene in the reaction product.

Table 1

| Cooling temperature (°C) | - 30 | - 20 | - 10 |
|---|---|---|---|
| 1,2,4,5-TriPEB purity (%) | 94.5 | 95.1 | 96.8 |
| 1,2,4,5-TriPEB yield (%) | 45.2 | 36.9 | 24.3 |
| Note: 1,2,4,5-TriPEB stands for 1-ethyl-2,4,5-triisopropylbenzene. | | | |

(2) Oxidation of Ethyltriisopropylbenzene

Pyromellitic anhydride was prepared in the same manner as in the above noted process (2) of Example 1 except that a crystallization product of 1-ethyl-2,4,5-triisopropylbenzene having a purity of 95.1 % obtained in the process (1) of Example 2 was used. As a result, pyromellitic anhydride was obtained in a yield of 18.0 % with respect to 1-ethyl-2,4,5-triisopropylbenzene in the first reaction product. The purity of this pyromellitic anhydride was 99.8 %.

COMPARATIVE EXAMPLE 1

(1) Preparation of Methyltriisopropylbenzene

A reaction was effected in the same manner as in the above noted process (1) of Example 1 except that propylene and toluene were used as starting materials instead of propylene and ethylbenzene. The reaction product was then subjected to quantitative analysis by gas chromatography. As a result, a mixture comprising 47.3 mol % of 1-methyl-2,4,5-triisopropylbenzene, 27.4 mol % of 1,2,3,5-methyltriisopropylbenzene, 1.2 mol % of toluene, 23.3 mol % of reaction intermediates such as 1-methyl-4-isopropylbenzene and 0.8 mol % of heavy fractions was obtained. The selectivity of 1-methyl-2,4,5-triisopropylbenzene was 63.3 %. The reaction product was then subjected to distillation at normal pressure in a boiling range of from 170 °C to 250 °C by means of an apparatus having 30 theoretical stages. The distillate was then subjected to quantitative analysis by gas chromatography. As a result, a mixture comprising 60.7 mol % of 1-methyl-2,4,5-triisopropylbenzene and 35.1 mol % of 1,2,3,5-methyltriisopropylbenzene was obtained.

(2) Oxidation of Methyltriisopropylbenzene

The distillate obtained in the above noted process (1) of Comparative Example 1 was used to prepare pyromellitic anhydride. Pyromellitic anhydride was prepared in the same manner as in process (2) of Example 1 except that the starting materials were changed. As a result, pyromellitic anhydride was obtained in a yield of 59.8 % with respect to 1-methyl-2,4,5-triisopropylbenzene in the reaction product. The purity of this pyromellitic anhydride was 63.6 %.

COMPARATIVE EXAMPLE 2

(1) Preparation of Tetraisopropylbenzene

A reaction was effected in the same manner as in process (1) of Example 1 except that propylene and cumene were used as starting materials instead of propylene and ethylbenzene. The reaction product was then subjected to quantitative analysis by gas chromatography. As a result, a mixture comprising 19.7 mol % of 1,2,4,5-tetraisopropylbenzene, 1.5 mol % of 1,2,3,5-tetraisopropylbenzene, 9.1 mol % of cumene, 69.1 mol % of reaction intermediates such as 1,4-diisopropylbenzene and 0.6 mol % of heavy fractions was obtained. The selectivity of 1,2,4,5-tetraisopropylbenzene was 92.9 %. The reaction product was then subjected to distillation at normal pressure in a boiling range of from 170 °C to 250 °C by means of an apparatus having 30 theoretical stages. The distillate was then subjected to quantitative analysis by gas chromatography. As a result, a mixture comprising 89.6 mol % of 1,2,4,5-tetraisopropylbenzene and 6.82 mol % of 1,2,3,5-tetraisopropylbenzene was obtained.

(2) Oxidation of Tetraisopropylbenzene

The distillate obtained in the process (1) of Comparative Example 2 was used to prepare pyromellitic anhydride. Pyromellitic anhydride was prepared in the same manner as in the process (2) of Example 1 except that the starting materials were changed. As a result, pyromellitic anhydride was obtained in a yield of 52.5 % with respect to 1,2,4,5-tetraisopropylbenzene in the reaction product. The purity of this pyromellitic anhydride was 93.9 %.

COMPARATIVE EXAMPLE 3

(1) Preparation of Tetramethylbenzene

A reaction was effected in the same manner as in process (1) of Example 1 except that methanol and p-xylene were used as starting materials instead of propylene and ethylbenzene, and the reaction temperature was 252 °C. The reaction product was then subjected to quantitative analysis by gas chromatography. As a result, a mixture comprising 42.1 mol % of 1,2,4,5-tetramethylbenzene (durene), 36.7 mol % of 1,2,3,5-tetramethylbenzene, 1.2 mol % of p-xylene, 13.1 mol % of reaction intermediates such as trimethylbenzene and 6.9 mol % of heavy fractions was obtained. The selectivity for 1,2,4,5-tetramethylbenzene was 53.4 %. The reaction product was subjected to distillation at normal pressure in a boiling range of from 170 °C to 250 °C by means of an apparatus having 30 theoretical stages. The distillate was then subjected to quantitative analysis by gas chromatography. As a result, a mixture comprising 52.1 mol % of 1,2,4,5-tetramethylbenzene and 45.4 mol % of 1,2,3,5-tetramethylbenzene was obtained.

(2) Oxidation of Tetramethylbenzene

The distillate obtained in the process (1) of Comparative Example 3 was used to prepare pyromellitic anhydride. Pyromellitic anhydride was prepared in the same manner as in process (2) of Example 1 except that the starting materials were changed. As a result, pyromellitic anhydride was obtained in a yield of 66.0 % with respect to 1,2,4,5-tetramethylbenzene in the reaction product. The purity of this pyromellitic anhydride was 66.5 %.

COMPARATIVE EXAMPLE 4

(1) Preparation of Dimethyldiisopropylbenzene

A reaction was effected in the same manner as in process (1) of Example 1 except that propylene and p-xylene were used as starting materials instead of propylene and ethylbenzene, and the reaction temperature was 107 °C. The reaction product was then subjected to quantitative analysis by gas chromatography. As a result, a mixture comprising 67.2 mol % of 1,4-dimethyl-2,5-diisopropylbenzene, 18.7 mol % of 1,2,3,5-dimethyldiisopropylbenzene, 1.3 mol % of p-xylene, 10.7 mol % of dimethylisopropylbenzene and 2.1 mol % of heavy fractions was obtained. The selectivity of 1,2,4,5-tetramethylbenzene was 80.1 %. The reaction product was then subjected to distillation under the same conditions as used in the process (1) of Example 1. The distillate was then subjected to quantitative analysis by gas chromatography. As a result, a mixture comprising 79.1 mol % of 1,4-dimethyl-2,5-diisopropylbenzene and 19.6 mol % of 1,2,3,5-dimethyldiisopropylbenzene was obtained.

(2) Oxidation of Dimethyldiisopropylbenzene

The distillate obtained in the process (1) of Comparative Example 4 was used to prepare pyromellitic anhydride. Pyromellitic anhydride was prepared in the same manner as in the process (2) of Example 1 except that the starting materials were changed. As a result, pyromellitic anhydride was obtained in a yield of 45.7 % with respect to 1,4-dimethyl-2,5-diisopropylbenzene in the reaction product. The purity of this pyromellitic anhydride was 83.1 %.

While the invention has been described in detail and with reference to specific embodiments thereof, it will be apparent to one skilled in the art that various changes and modifications can be made therein without departing from the spirit and scope thereof.

**Claims**

1.  A method for producing pyromellitic anhydride comprising the step of oxidizing 1-ethyl-2,4,5-triisopropylbenzene.

2.  The method for producing pyromellitic anhydride of Claim 1 further comprising the step of alkylating ethylbenzene with propylene to prepare said 1-ethyl-2,4,5,triisopropylbenzene.

3.  The method for producing pyromellitic anhydride of Claim 2, wherein said method comprises alkylating said 1-ethyl-2,4,5-triisopropylbenzene in the presence of a supported heteropolyacid or a salt thereof as an alkylation catalyst.

4. The method for producing pyromellitic anhydride of Claim 3, wherein said heteropolyacid or salt thereof is dodecatungstophosphoric acid, dodecatungstosilicic acid, dodecamolybdophosphoric acid, dodecatungstogermanic acid or dodecamolybdogermanic acid.

**European Patent Office**

# EUROPEAN SEARCH REPORT

Application Number

EP 95 11 6565

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | US-A-2 833 816 (SAFFER ET AL.)<br>* column 11, line 42 - line 43 *<br>* claims 1,11 *<br>--- | 1 | C07C63/313<br>C07C51/265<br>C07C15/02<br>C07C2/66 |
| Y | EP-A-0 163 851 (HÜLS AG)<br>* page 5, line 29 - line 36 *<br>* claim 1 *<br>--- | 1 | |
| Y | US-A-2 576 625 (MILLER)<br>* column 2, line 10 - line 29 *<br>* column 2, line 48 - line 52 *<br>* claim 1 *<br>--- | 1 | |
| X | CHEMICAL ABSTRACTS, vol. 69, no. 7, 12 August 1968, Columbus, Ohio, US; abstract no. 26873m, BABIN,E.P.ET AL. 'ALKYLATION OF ETHYLBENZENE BY PROPYLENE.' page 2491 ;column 2 ;<br>* abstract *<br>& KHIM.PROM, vol.9, no.43, 1967, DONETSK pages 667 - 669 BABIN,E.P. ET AL.<br>----- | 2 | **TECHNICAL FIELDS SEARCHED** (Int.Cl.6)<br><br>C07C |

The present search report has been drawn up for all claims

| Place of search | Date of completion of the search | Examiner |
|---|---|---|
| THE HAGUE | 26 January 1996 | Klag, M |